(19) Europäisches Patentamt
European Patent Office
Office européen des brevets

(11) **EP 4 497 746 A1**

(12) **EUROPEAN PATENT APPLICATION**
published in accordance with Art. 153(4) EPC


(43) Date of publication:
**29.01.2025 Bulletin 2025/05**

(21) Application number: **23775252.2**

(22) Date of filing: **21.03.2023**

(51) International Patent Classification (IPC):
***C07D 211/26** (2006.01)* ***A61K 9/51** (2006.01)*
***A61K 9/127** (2025.01)* ***A61K 47/22** (2006.01)*
***A61K 47/18** (2017.01)* ***C07D 295/13** (2006.01)*
***C07D 211/56** (2006.01)* ***C07D 233/61** (2006.01)*
***C07D 213/73** (2006.01)* ***C07D 233/60** (2006.01)*

(52) Cooperative Patent Classification (CPC):
**A61K 9/127; A61K 9/51; A61K 47/18; A61K 47/22; C07C 219/06; C07D 211/26; C07D 211/56; C07D 213/73; C07D 233/60; C07D 233/61; C07D 295/13**

(86) International application number:
**PCT/KR2023/003696**

(87) International publication number:
**WO 2023/182756 (28.09.2023 Gazette 2023/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **21.03.2022 KR 20220034692**
**20.03.2023 KR 20230035965**



(71) Applicant: **Medicibio Co., Ltd**
**Yongin-si, Gyeonggi-do 17095 (KR)**

(72) Inventor: **KI, Min Hyo**
**Yongin-si Gyeonggi-do 17095 (KR)**

(74) Representative: **Mewburn Ellis LLP**
**Aurora Building**
**Counterslip**
**Bristol BS1 6BX (GB)**


(54) **NOVEL IONIZABLE LIPID AND LIPID NANOPARTICLE COMPOSITION USING SAME**


(57) The present invention relates to a novel ionizable lipid and a lipid nanoparticle composition using same. It has been identified that the lipid nanoparticle composed of the novel ionizable lipid exhibits an excellent rate of gene encapsulation efficiency and of gene delivery efficacy in in vitro/in vivo system and, simultaneously, exhibits an excellent rate of gene delivery efficacy even in serum-free environments, and thus the present invention can be effectively used as a composition for drug delivery.


[FIG. 1]

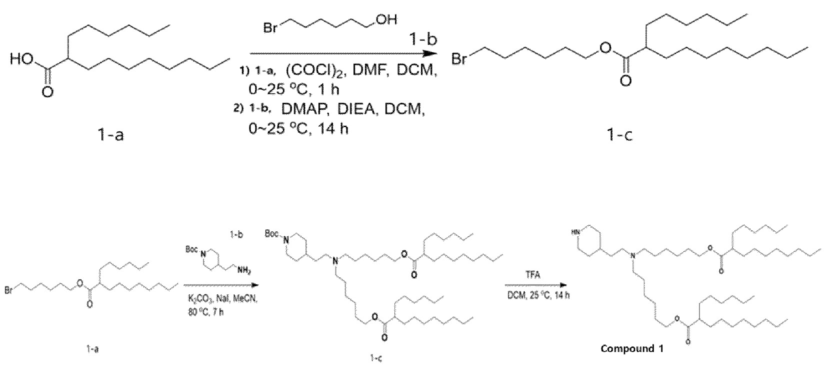





EP 4 497 746 A1

**Description**

Technical Field

The present invention relates to a novel ionizable lipid and a lipid nanoparticle composition using the same.

Background Art

Ever since the release of nucleic acid-based medicines more than 40 years ago to aid production of deficient proteins by injecting plasmid DNA in vivo, various types have been reported, such as antigen, decoy, antisense, siRNA, and miRNA that inhibit the transcription and translation of genes. Nucleic acid-based medicines have been attracting attention as personalized therapeutics through complementary binding with DNA or RNA with a specific sequence by targeting DNA or RNA rather than proteins. Nucleic acid-based medicines are utilized not only as therapeutic agents but also as prophylactic agents that defend against diseases by injecting genes capable of expressing antigens against specific diseases. Gene-based vaccines are divided into DNA vaccines, RNA vaccines, and viral vector vaccines, of which RNA vaccines take effects in a way that antigens are expressed in vivo by injecting antigen-encoding mRNA into the human body to induce formation of antibodies against the antigen, without potential risks of infection by viral vector-based vaccines or genetic mutations of DNA vaccines while ensuring rapid development, and thus have been in the spotlight as an effective countermeasure against COVID-19 that broke out in 2019.

However, nucleic acid-based drugs are easily degraded by nucleases in the human body and have shortcomings of not being easily delivered intracellularly due to being negatively charged macromolecules, such that it is necessary to have a stable and efficient way to deliver the drugs to a desired site. While delivery techniques based on various materials such as lipids, polymers, dendrimers, and inorganic metallic materials have been reported as delivery systems for nucleic acids, lipid nanoparticles have been used in patisiran, the new siRNA drug first approved by FDA in 2018, as well as mRNA vaccines against COVID-19 approved for emergency use in 2020. Current lipid nanoparticles are generally used in the form in which four constituents, including ionizable lipids, phospholipids (helper lipids), cholesterol (structural maintenance lipids), and PEG-lipids, are mixed in a certain ratio.

With the commercialization of new siRNA drugs and mRNA vaccines, there are various issues on the lipid nanoparticle-based delivery that need to be addressed, such as improvement in stability and cell permeability, which are challenges in the field of traditional nucleic acid drug delivery, as well as targeting, securing easiness in storage and distribution, mitigating side effects, reducing costs, and responding to breakthrough infections. Therefore, there is a need for the development of lipid nanoparticles that can solve new challenges in the nucleic acid drug market.

Disclosure of the Invention

Technical Goals

An object of the present invention is to provide a compound selected from a novel compound, or a pharmaceutically acceptable salt, tautomer, prodrug, or stereoisomer thereof.

Another object of the present invention is to provide a lipid nanoparticle composition including the compound.

Still another object of the present invention is to provide a composition for drug delivery, including the lipid nanoparticle composition; and a prophylactic or therapeutic agent.

Technical Solutions

To achieve the above objectives, the present invention provides a compound selected from a compound represented by the following Chemical Formula I, or a pharmaceutically acceptable salt, tautomer, prodrug, or stereoisomer thereof,

[Chemical Formula I]

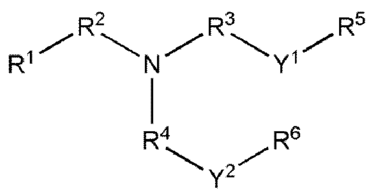

wherein, in the Chemical Formula I,
the $R^1$ may be selected from the group consisting of the following Chemical Formula I-1 to Chemical Formula I-3,

[Chemical Formula I-1]

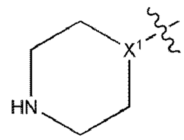

[Chemical Formula I-2]

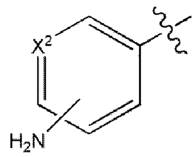

[Chemical Formula I-3]

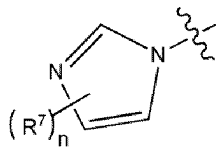

the $R^2$ may be selected from alkyl, alkenyl, or alkynyl of substituted or unsubstituted $C_{1-6}$, wherein the substitution is such that at least one $-CH_2-$ may be substituted with one selected from the group consisting of -O-, -C(=O)-, -OC(=O)-, -C(=O)O-, - OC(=O)O-, $-OP(=O)(OR^2)O-$, -OP(=O)(OH)O-, -OC(=S)-, -C(=S)O-, -SC(=O)-, -C(=O)S-, - SC(=S)-, -C(=S)S-, -SC(=O)O-, -OC(=O)S-, -SS-, -C(=O)NH-, -NHC(=O)-, $-C(=O)NR^2-$, - $NR^2C(=O)-$, -OC(=O)NH-, -NHC(=O)O-, $-OC(=O)NR^2-$, and $-NR^2C(=O)O-$,
the $R^3$ and $R^4$ may be independently selected from alkyl, alkenyl, or alkynyl of substituted or unsubstituted $C_{2-10}$, wherein the substitution is such that at least one $-CH_2-$ may be substituted with -CH(OH)-, -O-, or -SS-,
the $R^5$ and $R^6$ may be independently selected from alkyl, alkenyl, alkynyl, cycloalkyl, cycloalkenyl, cycloalkynyl, heterocyclyl, aryl, or heteroaryl of substituted or unsubstituted branched $C_{3-20}$, wherein the substitution is such that at least one $-CH_2-$ may be substituted with -CH(OH)-, -O-, or -SS-,
the $Y^1$ and $Y^2$ may be selected from -OC(=O)- or -C(=O)O-,
the $X^1$ and $X^2$ may be selected from CH or N,
the $R^7$ may be alkyl of $C_{1-6}$, and
n may be an integer of 1 to 2.

In addition, the present invention provides a lipid nanoparticle composition including the compound.
In addition, the present invention provides a composition for drug delivery, including the lipid nanoparticle composition; and a prophylactic or therapeutic agent. Advantageous Effects
According to the present invention, it was found that lipid nanoparticles by novel ionizable lipids exhibit an excellent gene encapsulation efficiency and superior gene delivery efficacy in in vitro/in vivo systems while exhibiting an outstanding gene delivery efficacy even in serum-free environment, such that the lipid nanoparticle may be useful as a composition for drug delivery.

Brief Description of Drawings

FIG. 1 shows a method of preparing ((2-(piperidin-4-yl)ethyl)azanediyl)bis(hexane-6,1-diyl) bis(2-hexyldecanoate).
FIG. 2 shows a method of preparing ((2-(piperazin-1-yl)ethyl)azanediyl)bis(hexane-6,1-diyl) bis(2-hexyldecanoate).
FIG. 3 shows a method of preparing ((4-aminobenzyl)azanediyl)bis(hexane-6,1-diyl) bis(2-hexyldecanoate).

FIG. 4 shows a method of preparing ((2-(6-aminopyridin-3-yl)ethyl)azanediyl)bis(hexane-6,1-diyl) bis(2-hexyldecanoate).

FIG. 5 shows a method of preparing ((3-(2-methyl-1H-imidazol-1-yl)propyl)azanediyl)bis(hexane-6,1-diyl) bis(2-hexyldecanoate).

FIG. 6 shows a method of preparing ((3-(2,4-dimethyl-1H-imidazol-1-yl)propyl)azanediyl)bis(hexane-6,1-diyl)bis(2-hexyldecanoate).

FIG. 7 shows a method of preparing heptadecan-9-yl 8-((3-(2,4-dimethyl-1H-imidazol-1-yl)propyl)(8-(nonyloxy)-8-oxooctyl)amino)octanoate.

FIG. 8 shows a method of preparing heptadecan-9-yl 8-((3-(2,4-dimethyl-1H-imidazol-1-yl)propyl)(8-oxo-8-(pentan-2-yloxy)octyl)amino)octanoate.

Best Mode for Carrying Out the Invention

Hereinafter, the present invention will be described in detail.

The present invention provides a compound selected from a compound represented by the following Chemical Formula I, or a pharmaceutically acceptable salt, tautomer, prodrug, or stereoisomer thereof.

[Chemical Formula I]

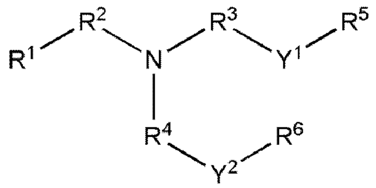

In the Chemical Formula I,

the $R^1$ may be selected from the group consisting of the following Chemical Formula I-1 to Chemical Formula I-3,

[Chemical Formula I-1]

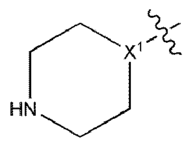

[Chemical Formula I-2]

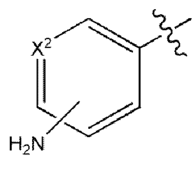

[Chemical Formula I-3]

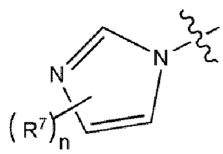

the $R^2$ may be selected from alkyl, alkenyl, or alkynyl of substituted or unsubstituted $C_{1-6}$, wherein the substitution is such that at least one $-CH_2-$ may be substituted with one selected from the group consisting of -O-, -C(=O)-, -OC(=O)-,

-C(=O)O-, - OC(=O)O-, -OP(=O)($OR^2$)O-, -OP(=O)(OH)O-, -OC(=S)-, -C(=S)O-, -SC(=O)-, -C(=O)S-, - SC(=S)-, -C(=S)S-, -SC(=O)O-, -OC(=O)S-, -SS-, -C(=O)NH-, -NHC(=O)-, -C(=O)$NR^2$-, - $NR^2$C(=O)-, -OC(=O)NH-, -NHC(=O)O-, -OC(=O)$NR^2$-, and -$NR^2$C(=O)O-,
the $R^3$ and $R^4$ may be independently selected from alkyl, alkenyl, or alkynyl of substituted or unsubstituted $C_{2-10}$, wherein the substitution is such that at least one -$CH_2$- may be substituted with -CH(OH)-, -O-, or -SS-,
the $R^5$ and $R^6$ may be independently selected from alkyl, alkenyl, alkynyl, cycloalkyl, cycloalkenyl, cycloalkynyl, heterocyclyl, aryl, or heteroaryl of substituted or unsubstituted branched $C_{3-20}$, wherein the substitution is such that at least one -$CH_2$- may be substituted with -CH(OH)-, -O-, or -SS-,
the $Y^1$ and $Y^2$ may be selected from -OC(=O)- or -C(=O)O-,
the $X^1$ and $X^2$ may be selected from CH or N,
the $R^7$ may be alkyl of $C_{1-6}$, and
n may be an integer of 1 to 2.

Alternatively, in the Chemical Formula I,

the $R^1$ may be a compound selected from the group consisting of the following Chemical Formulas I-4 to I-9.

[Chemical Formula I-4]

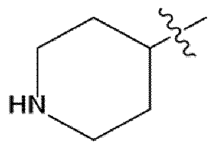

[Chemical Formula I-5]

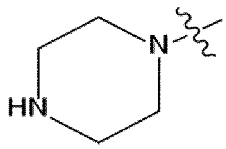

[Chemical Formula I-6]

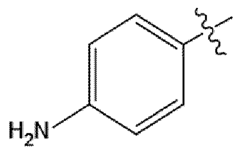

[Chemical Formula I-7]

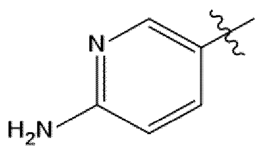

[Chemical Formula I-8]

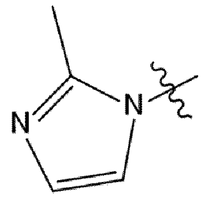

[Chemical Formula I-9]

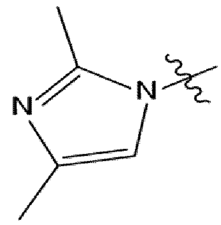

The $R^2$ may be alkyl of substituted or unsubstituted $C_{1-6}$, wherein the substitution is such that at least one $-CH_2-$ may be substituted with one selected from the group consisting of -O-, -C(=O)-, -OC(=O)-, -C(=O)O-, and -SS-.

The $R^3$ and $R^4$ may be alkyl of independently substituted or unsubstituted $C_{2-10}$, wherein the substitution is such that at least one $-CH_2-$ may be substituted with -CH(OH)-, - O-, or -SS-.

The $R^5$ and $R^6$ may be independently alkyl or alkenyl of substituted or unsubstituted branched $C_{3-20}$, wherein the substitution is such that at least one $-CH_2-$ may be substituted with -CH(OH)-, -O-, or -SS-.

The $Y^1$ and $Y^2$ may be selected from -OC(=O)- or -C(=O)O-.

Alternatively, in the Chemical Formula I,

the $R^1$ may be selected from the group consisting of the following Chemical Formulas I-4 to I-9, $R^2$ may be alkyl of substituted or unsubstituted $C_{1-6}$ wherein the substitution is such that at least one $-CH_2-$ may be substituted with one selected from the group consisting of -O-, -C(=O)-, -OC(=O)-, -C(=O)O-, and -SS-, the $R^3$ and $R^4$ may be independently alkyl of substituted or unsubstituted $C_{2-10}$ wherein the substitution is such that at least one $-CH_2-$ may be substituted with -CH(OH)-, -O-, or -SS-, the $R^5$ and $R^6$ may be independently alkyl or alkenyl of substituted or unsubstituted branched $C_{3-20}$ wherein the substitution is such that at least one $-CH_2-$ may be substituted with -CH(OH)-, -O-, or -SS-, and the $Y^1$ and $Y^2$ may be selected from -OC(=O)- or -C(=O)O-.

[Chemical Formula I-4]

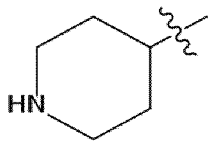

[Chemical Formula I-5]

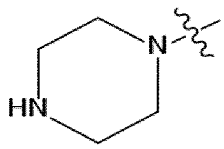

[Chemical Formula I-6]

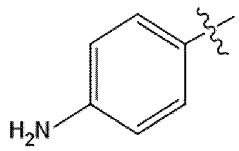

[Chemical Formula I-7]

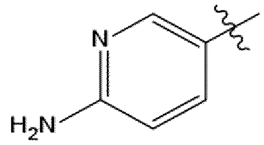

[Chemical Formula I-8]

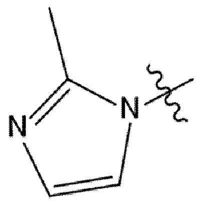

[Chemical Formula I-9]

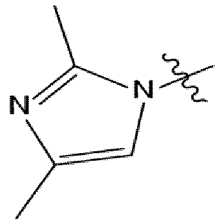

The compound may be one or more selected from the group consisting of, but is not limited to, ((2-(piperidin-4-yl)ethyl)azanediyl)bis(hexane-6,1-diyl) bis(2-hexyldecanoate); ((2-(piperazin-1-yl)ethyl)azanediyl)bis(hexane-6,1-diyl) bis(2-hexyldecanoate); ((4-aminobenzyl)azanediyl)bis(hexane-6,1-diyl) bis(2-hexyldecanoate); ((2-(6-aminopyridin-3-yl)ethyl)azanediyl)bis(hexane-6,1-diyl) bis(2-hexyldecanoate); ((3-(2-methyl-1H-imidazol-1-yl)propyl)azanediyl)bis(hexane-6,1-diyl) bis(2-hexyldecanoate); ((3-(2,4-dimethyl-1H-imidazol-1-yl)propyl)azanediyl)bis(hexane-6,1-diyl) bis(2-hexyldecanoate); heptadecan-9-yl 8-((3-(2,4-dimethyl-1H-imidazol-1-yl)propyl)(8-(nonyloxy)-8-oxooctyl)amino)octanoate; heptadecan-9-yl 8-((3-(2,4-dimethyl-1H-imidazol-1-yl)propyl)(8-oxo-8-(pentan-2-yloxy)octyl)amino)octanoate; bis(2-hexyldecyl) 7,7'-((2-(6-aminopyridin-3-yl)ethyl)azanediyl) diheptanoate; 6-((2-(6-aminopyridin-3-yl)ethyl)(8-(nonanoyloxy)octyl)amino)hexyl 2-hexyldecanoate; ((2-(6-aminopyridin-3-yl)ethyl)azanediyl)bis(hexane-6,1-diyl) bis(2-(2-(butyldisulfanyl)ethyl)octanoate); ((((3-(2-methyl-1H-imidazol-1-yl)propyl)azanediyl)bis(ethane-2,1-diyl))bis(oxy))bis(propane-3,1-diyl) bis(2-hexyldecanoate); heptadecan-9-yl 8-((3-(2-methyl-1H-imidazol-1-yl)propyl)(8-(nonyloxy)-8-oxooctyl)amino)octanoate; and ((3-((2,4-dimethyl-1H-imidazol-1-yl)methoxy)propyl)azanediyl) bis(hexane-6,1-diyl) bis(2-hexyldecanoate).

Specifically, the ((2-(piperidin-4-yl)ethyl)azanediyl)bis(hexane-6,1-diyl) bis(2-hexyldecanoate) (hereinafter referred to as Compound 1) has the same structure as the following Chemical Formula 1, with a molecular formula of $C_{51}H_{100}N_2O_4$ and a molecular weight of 805.35.

[Chemical Formula 1]

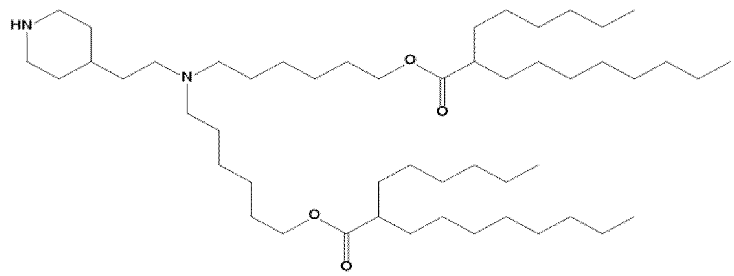

The ((2-(piperazin-1-yl)ethyl)azanediyl)bis(hexane-6,1-diyl) bis(2-hexyldecanoate) (hereinafter referred to as Compound 2) has the same structure as the following Chemical Formula 2, with a molecular formula of $C_{50}H_{99}N_3O_4$ and a molecular weight of 806.34.

[Chemical Formula 2]

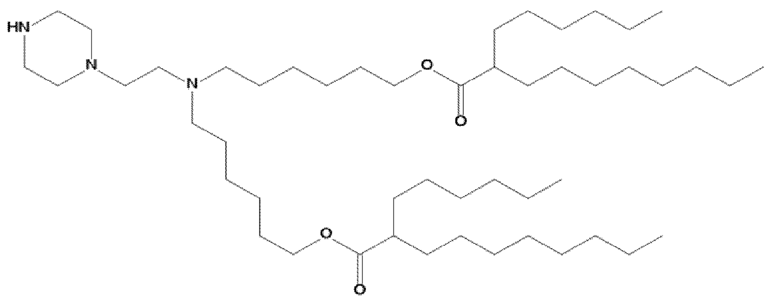

The ((4-aminobenzyl)azanediyl)bis(hexane-6,1-diyl) bis(2-hexyldecanoate) (hereinafter referred to as Compound 3) has the same structure as the following Chemical Formula 3, with a molecular formula of $C_{51}H_{94}N_2O_4$ and a molecular weight of 799.30.

[Chemical Formula 3]

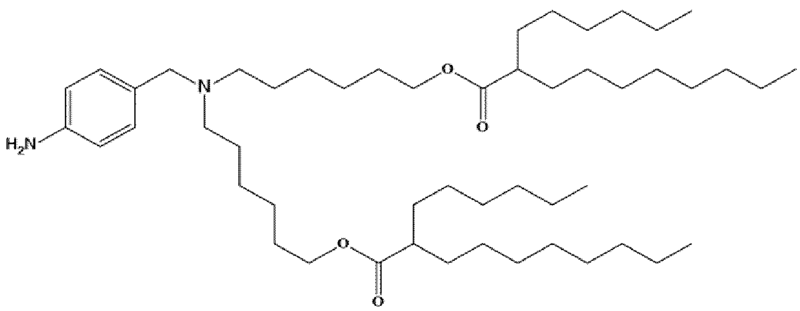

The ((2-(6-aminopyridin-3-yl)ethyl)azanediyl)bis(hexane-6,1-diyl) bis(2-hexyldecanoate) (hereinafter referred to as Compound 4) has the same structure as the following Chemical Formula 4, with a molecular formula of $C_{51}H_{95}N_3O_4$ and a molecular weight of 814.32.

[Chemical Formula 4]

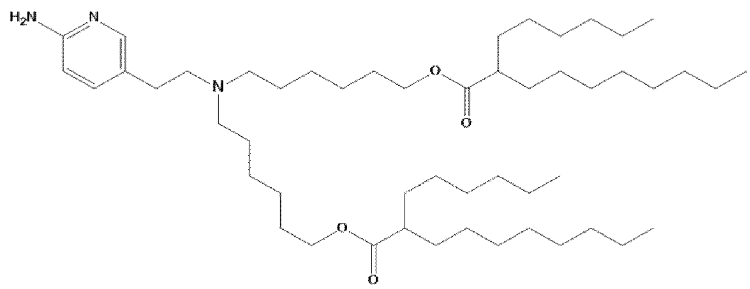

The ((3-(2-methyl-1H-imidazol-1-yl)propyl)azanediyl)bis(hexane-6,1-diyl) bis(2-hexyldecanoate) (hereinafter referred to as Compound 5) has the same structure as the following Chemical Formula 5, with a molecular formula of $C_{51}H_{97}N_3O_4$ and a molecular weight of 816.33.

[Chemical Formula 5]

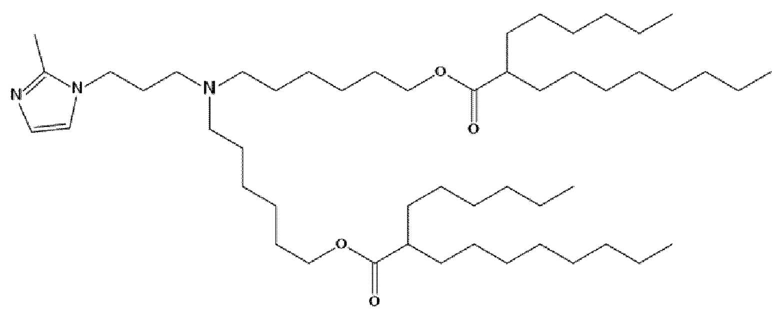

The ((3-(2,4-dimethyl-1H-imidazol-1-yl)propyl)azanediyl)bis(hexane-6,1-diyl)bis(2-hexyldecanoate) (hereinafter referred to as Compound 6) has the same structure as the following Chemical Formula 6, with a molecular formula of $C_{52}H_{99}N_3O_4$ and a molecular weight of 830.36.

[Chemical Formula 6]

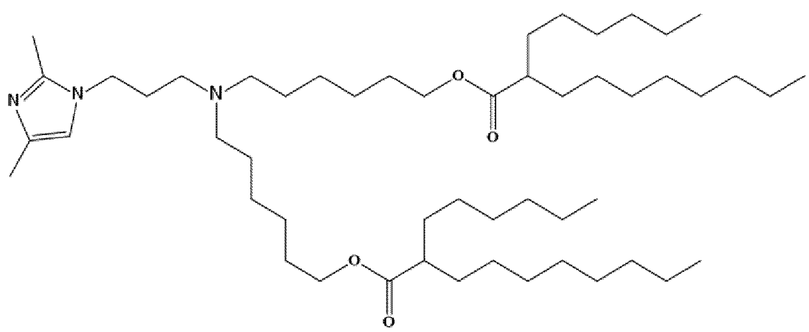

The heptadecan-9-yl 8-((3-(2,4-dimethyl-1H-imidazol-1-yl)propyl)(8-(nonyloxy)-8-oxooctyl)amino)octanoate (hereinafter referred to as Compound 7) has the same structure as the following Chemical Formula 7, with a molecular formula of $C_{50}H_{95}N_3O_4$ and a molecular weight of 802.31.

[Chemical Formula 7]

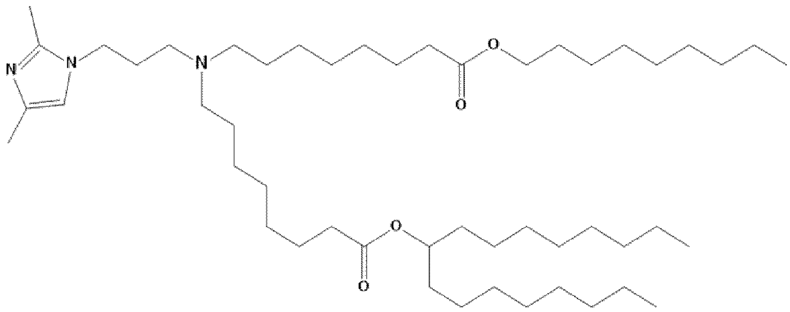

The heptadecan-9-yl 8-((3-(2,4-dimethyl-1H-imidazol-1-yl)propyl)(8-oxo-8-(pentan-2-yloxy)octyl)amino)octanoate (hereinafter referred to as Compound 8) has the same structure as the following Chemical Formula 8, with a molecular formula of $C_{46}H_{87}N_3O_4$ and a molecular weight of 746.20.

[Chemical Formula 8]

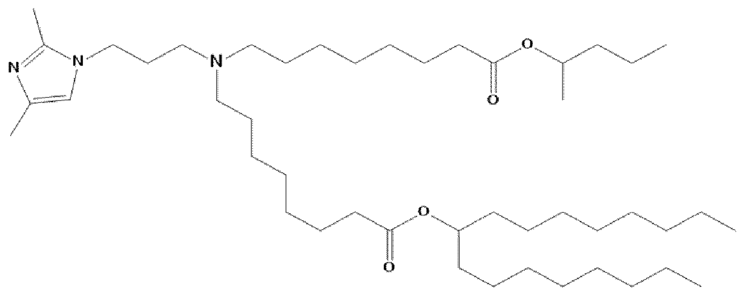

The bis(2-pentyloctyl)8,8'-((2-(piperidin-4-yl)ethyl)azanediyl) dioctanoate (hereinafter referred to as Compound 9) has the same structure as the following Chemical Formula 9, with a molecular formula of $C_{49}H_{96}N_2O_4$ and a molecular weight of 777.30.

[Chemical Formula 9]

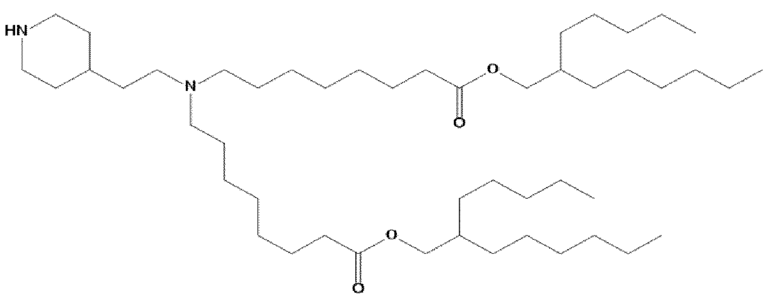

The bis(2-hexyldecyl) 7,7'-((2-(6-aminopyridin-3-yl)ethyl)azanediyl) diheptanoate (hereinafter referred to as Compound 10) has the same structure as the following Chemical Formula 10, with a molecular formula of $C_{53}H_{99}N_3O_4$ and a molecular weight of 842.37.

[Chemical Formula 10]

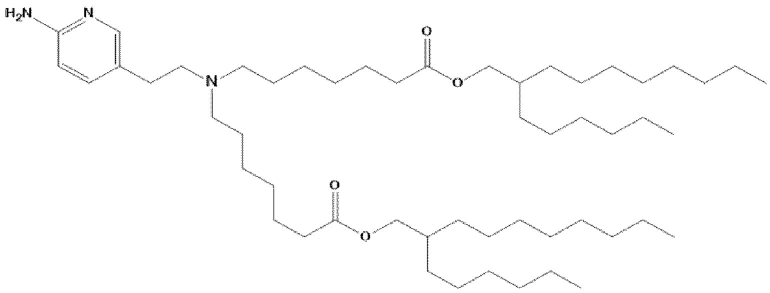

The 6-((2-(6-aminopyridin-3-yl)ethyl)(8-(nonanoyloxy)octyl)amino)hexyl 2-hexyldecanoate (hereinafter referred to as Compound 11) has the same structure as the following Chemical Formula 11, with a molecular formula of $C_{46}H_{85}N_3O_4$ and a molecular weight of 744.18.

[Chemical Formula 11]

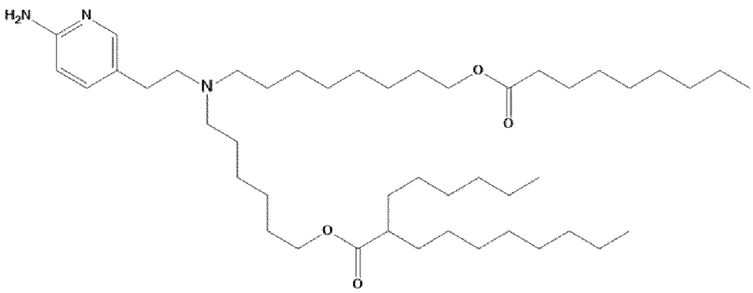

The ((2-(6-aminopyridin-3-yl)ethyl)azanediyl)bis(hexane-6,1-diyl) bis(2-(2-(butyldisulfanyl)ethyl)octanoate (hereinafter referred to as Compound 12) has the same structure as the following Chemical Formula 12, with a molecular formula of $C_{47}H_{87}N_3O_4S_4$ and a molecular weight of 886.47.

[Chemical Formula 12]

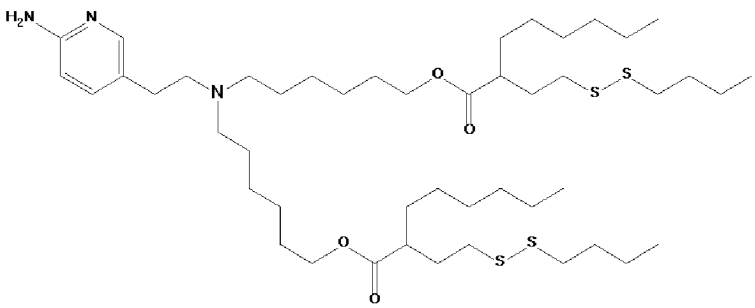

The ((((3-(2-methyl-1H-imidazol-1-yl)propyl)azanediyl)bis(ethane-2,1-diyl))bis(oxy))bis(propane-3,1-diyl) bis(2-hexyldecanoate) (hereinafter referred to as Compound 13) has the same structure as the following Chemical Formula 13, with a molecular formula of $C_{49}H_{93}N_3O_6$ and a molecular weight of 820.28.

[Chemical Formula 13]

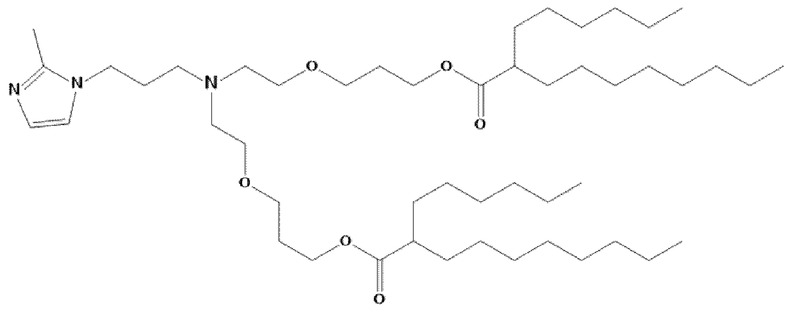

The heptadecan-9-yl 8-((3-(2-methyl-1H-imidazol-1-yl)propyl)(8-(nonyloxy)-8-oxooctyl)amino)octanoate (hereinafter referred to as Compound 14) has the same structure as the following Chemical Formula 14, with a molecular formula of $C_{49}H_{93}N_3O_4$ and a molecular weight of 788.28.

[Chemical Formula 14]

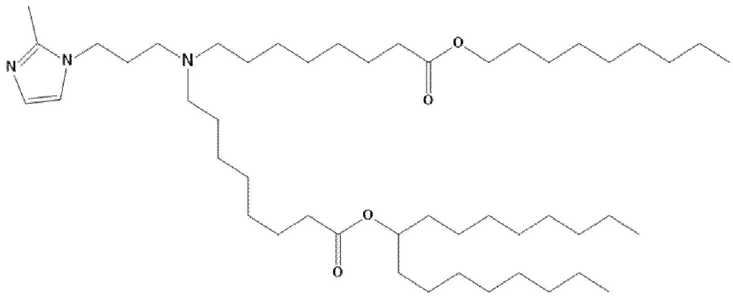

The ((3-((2,4-dimethyl-1H-imidazol-1-yl)methoxy)propyl)azanediyl) bis(hexane-6,1-diyl) bis(2-hexyldecanoate) (hereinafter referred to as Compound 15) has the same structure as the following Chemical Formula 15, with a molecular formula of $C_{53}H_{101}N_3O_5$ and a molecular weight of 860.39.

[Chemical Formula 15]

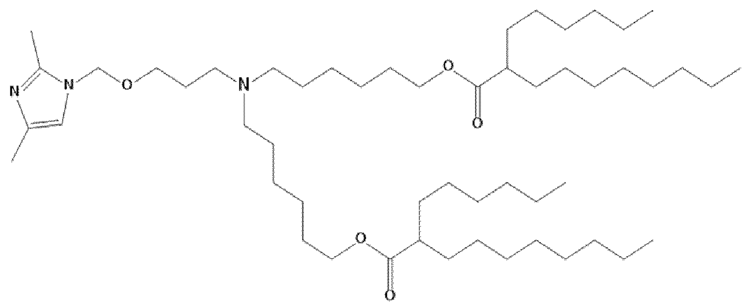

In addition, the present invention provides a lipid nanoparticle composition including the compound.
The compound may be an ionizable lipid.
The lipid nanoparticles have excellent safety and stability and may exhibit a function as a delivery to allow a gene including RNA, DNA, or a hybrid type thereof as an active ingredient to derive its effectiveness well in cells.
The lipid nanoparticle composition may include, but is not limited to, one or more selected from the group consisting of a neutral lipid, steroid, and polymeric polylipid.
The neutral lipid may be a phospholipid or a glycolipid. Specifically, the phospholipid may be one or more selected from the group consisting of dioleoylphosphatidylethanolamine (DOPE), distearoylphosphatidylcholine (DSPC), palmitoyloleoylphosphatidylcholine (POPC), egg phosphatidylcholine (EPC), dioleoylphosphatidylcholine (DOPC), dipalmitoylphosphatidylcholine (DPPC), dioleoylphosphatidylglycerol (DOPG), dipalmitoylphosphatidylglycerol (DPPG), distearoylphosphatidylethanolamine (DSPE), phosphatidylethanolamine (PE), and dipalmitoylphosphatidylethanolamine, and the glycolipid may be one or more selected from the group consisting of glucosylceramide, galactosylceramide, glucosylsphingosine, galactosyl sphingosine, and phosphoglycoceramide, but are not limited thereto.

The steroid may be, but is not limited to, one or more selected from the group consisting of cholesterol, bile acid derivatives, and cholinic acid derivatives.

The polymeric polylipid may be a pegylated lipid with a structure in which water-soluble polymers and lipids are bonded, and the pegylated lipid may be one or more selected from the group consisting of PEG (PEG-DAA) bound to a dialkyloxypropyl; PEG-c-DOMG and PEG-DMG as PEG (PEG-DAG) bound to diacylglycerol; PEG-DLPE, PEG-DMPE, and PEG-DSPE as PEG (PEG-PE) bound to phospholipids such as phosphatidylethanolamine; PEG (PEG-CER) conjugated to ceramides; PEG conjugated to cholesterol or derivatives thereof; PEG-modified phosphatidic acid; PEG-modified dialkylamine; and PEG-modified dialkylglycerol, but are not limited thereto. In addition, the pegylated lipid may be those (functionalized PEGs) having a functional group bound to a side that is not bound with lipids. The available functional groups may include, but are not limited to, one or more selected from the group consisting of succinyl, carboxylic acid, maleimide, n-hydroxysuccinimide, amine group, biotin, cyanur group, and folate.

The ionizable lipid may be prepared in a form of lipid nanoparticles, including neutral lipids, steroids, and polymeric polylipids. For a composition suitable for the preparation of lipid nanoparticles, 20 to 65 mol% of ionizable lipids, 2.5 to 30 mol% of neutral lipids, 20 to 60 mol% of steroids, and 0.5 to 5 mol% of polymeric polylipids may be included in a lipid composition excluding active ingredients. For example, ionizable lipids:neutral lipids:steroids:polymeric polylipids may be composed in a mol% ratio selected from the group consisting of 60:5:33.5:1.5, 50:10:38.5:1.5, 40:15:43.5:1.5, 30:20:48.5:1.5, 25:25:48.5:1.5, 60:5:32:3 or 50:10:37:3, 40:15:42:3, 30:20:47:3, and 25:25:47:3.

In addition, the composition may further include prophylactic or therapeutic agents, and the agent may be a gene including a single-stranded or double-stranded RNA, DNA, or a mixture thereof, specifically it may be one or more selected from the group consisting of, but is not limited to, small interfering RNA (siRNA), ribosomal ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), anti-sense oligonucleotide, small hairpin ribonucleic acid (shRNA), microribonucleic acid (miRNA), asymmetric interfering ribonucleic acid (aiRNA), dicer-substrate ribonucleic acid (dsRNA), ribozyme, peptide nucleic acid (PNA), deoxyribozyme (DNAzyme), and guide ribonucleic acid (sgRNA) for gene editing. In addition, the active ingredient of the composition may be various negatively charged peptides, protein drugs, protein-nucleic acid structures or hyaluronic acid-peptide conjugates, hyaluronic acid-protein conjugates, and anionic biopolymer-drug conjugates such as antibodies.

The lipid nanoparticle composition of the present invention, including ionizable lipids, neutral lipids, steroids, and polymeric polylipids, may be dissolved in solvents that are miscible with ethanol or water to be prepared as lipid solutions. Separately, the active ingredient may be dissolved in citric acid or acetic acid buffer in a pH of 4.0±1.0 to be prepared as an active ingredient solution. In addition, the lipid solution and the active ingredient solution may be mixed by a microfluidic mixing device (Benchtop Nanoassemblr, Precision Nanosystems) at a volume ratio of 1:3 and a flow rate of about 10~15 mL/min to be prepared into lipid nanoparticles.

In addition, the present invention provides a composition for drug delivery, including the lipid nanoparticle composition; and a prophylactic or therapeutic agent.

The composition for drug delivery may be administered via various routes, including parenteral administration to mammals including humans, wherein the parenteral administration may be applied intravenously, subcutaneously, intraperitoneally, or locally, and the dosage may vary depending on the patient's condition and weight, severity of diseases, drug form, and route and time of administration, wherein the dosage may be appropriately selected by those skilled in the art.

When preparing the composition for drug delivery according to an example embodiment, the composition is prepared using a diluent or excipient such as commonly used fillers, extenders, freeze-drying agents, binders, wetting agents, disintegrating agents, and surfactants.

Preparations for parenteral administration include sterile aqueous solutions, non-aqueous solvents, suspended solvents, emulsions, freeze-drying agents, and suppositories.

As the non-aqueous solvent and suspension solvent, propylene glycol, polyethylene glycol, vegetable oils such as olive oil, and injectable esters such as ethyl oleate may be used. As the base of the suppository, witepsol, macrogol, Tween 61, cacao butter, laurin butter, glycerol, and gelatin may be used.

The composition for drug delivery of the present invention may be administered with inclusion of a pharmaceutically effective amount of prophylactic or therapeutic agents. The effective dose level of the prophylactic or therapeutic agent may be determined according to the patient's disease type, severity, activity of the drug, sensitivity to the drug, time of administration, route of administration and excretion rate, duration of treatment, elements including concomitant drugs and other well-known factors in the field of medicine. The composition according to an example embodiment may be administered as individual therapeutics or in combination with other therapeutics, administered sequentially or simultaneously with the conventional therapeutics, and administered in single or multiple does. Considering all the factors above, it is important to administer an amount that is able to derive the maximum effect with a minimum amount without side effects, which may be easily determined by those skilled in the art. For example, the composition may be administered in an amount of 0.01 to 100 mg/kg, 0.1 to 50 mg/kg, or 1 to 10 mg/kg.

The present invention may be described in more detail through the following specific examples and experimental examples, but the present invention is not limited to the following examples.

Modes for Carrying Out the Invention

Hereinafter, the present invention will be described in more detail through examples to help understanding of the present invention. However, examples below are merely intended to illustrate the content of present invention, and the scope of the present invention is not limited to the following examples. Examples of the present invention are provided to more completely explain the present invention to those skilled in the art.

[Example 1] Preparation of Compounds

1-1. Preparation of Compound 1

As shown in FIG. 1, Compound 1-a (25.0 g, 97.5 mmol) was dissolved in dichloromethane (hereinafter referred to as DCM, 250 mL), and then dimethylformamide (356 mg, 4.87 mmol) and oxalyl chloride (61.9 g, 487 mmol) were added at 0°C and stirred at 25°C for 1 hour. The mixture was then subjected to the primary concentration under reduced pressure, dissolved in toluene (50 mL×2 times), followed by the secondary concentration under reduced pressure to obtain 2-hexyldecanoyl chloride (26.8 g, crude) in a yellow oil form. Compound 1-b (26.5 g, 146 mmol) was dissolved in DCM (200 mL), dimethylaminopyridine (hereinafter referred to as DMAP, 1.19 g, 9.75 mmol) and diisopropylethylamine (hereinafter referred to as DIEA, 75.6 g, 585 mmol) were then added, and the temperature was lowered to 0°C, followed by slow addition of 2-hexyldecanoyl chloride (26.8 g, 97.5 mmol) dissolved in DCM (150 mL). The temperature of the mixture was slowly elevated to 25°C, and the mixture was stirred for 14 hours. Afterwards, it was washed with hydrochloric acid solution (0.1M, 400 mL×2 times) and saturated sodium bicarbonate solution (400 mL×2 times), the organic layer was dried with sodium sulfate (hereinafter referred to as $Na_2SO_4$), concentration was performed under reduced pressure, and purification was conducted on silica gel (petroleum ether (hereinafter referred to as PE)/ethyl acetate (hereinafter referred to as EtOAc) = 100/1 to 49/1) to obtain Compound 1-c in a colorless oil form.

In acetonitrile (hereinafter referred to as MeCN, 40 mL), a mixture of Compound 1-a (3.00 g, 7.15 mmol), Compound 1-b (544 mg, 2.38 mmol), potassium carbonate (hereinafter referred to as $K_2CO_3$, 988 mg, 7.15 mmol), and sodium iodide (hereinafter referred to as NaI, 357 mg, 2.38 mmol) was stirred at 80°C for 7 hours. After that, it was diluted with purified water (80 mL), extracted with DCM (80 mL×3 times), washed with saturated sodium chloride solution (hereinafter referred to as brine, 150 mL), and dried with $Na_2SO_4$, followed by filtration. After concentration of the filtrate under reduced pressure, DCM (5 mL) and trifluoroacetic acid (hereinafter referred to as TFA, 5 mL) were added to the Compound 1-c (500 mg, 0.552 mmol) obtained by separating on the silica gel (PE/EtOAc = 9/1 to 2/1) and then stirred at 25°C for 14 hours. After that, it was concentrated under reduced pressure, added with DCM (20 mL) and purified water (20 mL), and adjusted to pH 11 using sodium hydroxide solution (hereinafter referred to as aq.NaOH, 1M), followed by extraction with DCM (30 mL×2 times). Afterwards, it was dried with $Na_2SO_4$, filtered, and concentrated under reduced pressure to obtain Compound 1 in a light yellow oil form, and the result of the $^1H$ NMR analysis for Compound 1 is as shown below.

$^1H$ NMR(400MHz, $CDCl_3$) $\delta$ 4.07(t, J = 6.8Hz, 4H), 3.05-3.08(m, 2H), 2.55-2.62(m, 2H), 2.28-2.44(m, 8H), 1.56-1.68(m, 10H), 1.26-1.45(m, 59H), 1.09-1.15(m, 2H), 0.86-0.89(m, 12H)

1-2. Preparation of Compound 2

As shown in FIG. 2, a mixture of Compound 2-a (3.00 g, 7.15 mmol), Compound 2-b (547 mg, 2.38 mmol), $K_2CO_3$ (988 mg, 7.15 mmol), and NaI (179 mg, 1.19 mmol) was stirred in MeCN (40 mL) at 80°C in the presence of nitrogen gas for 5 hours. Afterwards, it was diluted with purified water (80 mL), extracted with DCM (80 mL×3 times), washed with brine (150 mL), and dried with $Na_2SO_4$, followed by filtration. The filtrate was concentrated under reduced pressure, and DCM (5 mL) and TFA (7.70 g) were added to Compound 1-c (500 mg, 0.552 mmol) in a yellow oil form obtained by purifying on the silica gel (PE/EtOAc = 100/1 to 2/1), followed by stirring at 25°C for 14 hours. After that, it was concentrated under reduced pressure, added with DCM (20 mL) and purified water (20 mL), adjusted to pH 11 using aq.NaOH(1M), and extracted with DCM (30 mL×2 times), followed by drying with $Na_2SO_4$ and concentration under reduced pressure to obtain Compound 2 in a yellow oil form. The result of $^1H$ NMR analysis for Compound 2 is as shown below.

$^1H$ NMR(400MHz, $CDCl_3$) $\delta$ 4.06(t, J = 6.4Hz, 4H), 2.90(t, J = 4.8Hz, 4H), 2.57-2.61(m, 2H), 2.43-2.46(m, 8H), 2.30-2.31(m, 2H), 1.58-1.66(m, 8H), 1.26-1.47(m, 58 H), 0.88(t, J = 6.4Hz, 12 H)

1-3. Preparation of Compound 3

As shown in FIG. 3, Compound 3-a (25.0 g, 97.5 mmol) was added to DCM (250 mL), and dimethylformamide (356 mg, 4.87 mmol) and oxalyl chloride (61.9 g, 487 mmol) were added at 0°C, followed by stirring at 25°C for 1 hour. Thereafter, it was concentrated under reduced pressure, dissolved in toluene (50 mL×2), and then concentrated under reduced pressure to obtain 2-hexyldecanoyl chloride in a yellow oil form. Compound 3-b (26.5 g, 146 mmol) was added to DCM (200 mL), DMPA (1.19 g, 9.75 mmol) and DIEA (75.6 g, 585 mmol) were added, the temperature was lowered to 0°C, and then 2-hexyldecanoyl chloride (26.8 g, 97.5 mmol) dissolved in DCM (150 mL) was slowly added. The temperature of the mixture was slowly elevated to 25°C, followed by stirring for 14 hours. Afterwards, it was washed with hydrochloric acid solution (0.1M, 400 mL×2 times) and saturated sodium bicarbonate solution (400 mL×2 times), the organic layer was dried with $Na_2SO_4$, and after concentration under reduced pressure, purification was performed on silica gel (PE/EtOAc = 100/1 to 49/1) to obtain Compound 1-c in a colorless oil form. Compounds 3-c (3 g, 7.15 mmol) and 3-d (530 mg, 2.38 mmol), $K_2CO_3$ (988 mg, 7.15 mmol), and NaI (179 mg, 1.19 mmol) were added to MeCN (40 mL) and stirred at 80°C for 6 hours. Subsequently, it was diluted with purified water (60 mL), extracted with DCM (60 mL×2 times), dried with $Na_2SO_4$, concentrated under reduced pressure, and purified on silica gel (PE/EtOAc = 100/1 to 6/1) to obtain Compound 3-e in a colorless semi-solid form. Compound 3-e (600 mg, 0.667 mmol) was added to DCM (6 mL), and then TFA (6 mL) was added, followed by stirring at 25°C for 16 hours. Afterwards, it was concentrated under reduced pressure, added with DCM (20 mL) and purified water (20 mL), and then adjusted to pH 9 using saturated sodium carbonate solution. It was extracted with DCM (30 mL×2 times), dried with $Na_2SO_4$, and concentrated under reduced pressure to obtain Compound 3 in a yellow oil form. The result of $^1$H NMR analysis for Compound 3 is as shown below.

$^1$H NMR(400MHz, $CDCl_3$) δ 7.07-7.09(m, 2H), 6.63-6.65(m, 2H), 4.05(t, J = 6.8Hz, 4H), 3.62(s, 2H), 3.44(s, 2H), 2.29-2.38(m, 6H), 1.56-1.62(m, 8H), 1.40-1.45(m, 8H), 1.26-1.31(m, 48H), 0.86-0.90(m, 12H).

1-4. Preparation of Compound 4

As shown in FIG. 4, a mixture of toluene (20 mL) and purified water (5 mL) was added with Compound 4-a (1.03 g, 5.08 mmol), Compound 4-b (850 mg, 3.39 mmol), cesium carbonate (3.31 g, 10.2 mmol), and palladium catalyst (Pd(dppf) $Cl_2.CH_2Cl_2$, 276 mg, 0.339 mmol), followed by stirring in the presence of nitrogen gas at 80°C for 12 hours. Afterwards, it was diluted with purified water (40 mL), extracted with EtOAc (40 mL×3 times), and concentrated under reduced pressure, and Compound 4-c (640 mg, 2.39 mmol) in a yellow solid form obtained by purification on silica gel (PE/EtOAc = 100/1 to 1/1) was added to DCM (6 mL) and TFA (6 mL) and stirred at 25°C for 2 hours. It was then concentrated under reduced pressure, diluted with DCM (20 mL) and purified water (20 mL), and adjusted to pH 11 using aq.NaOH (1M), followed by extraction with a DCM/methanol (hereinafter referred to as MeOH) mixture solution (10/1, 30 mL×4 times). The extract was dried with $Na_2SO_4$ and concentrated under reduced pressure to obtain Compound 4-d in a yellow oil form. Compound 4-d (3.10 g, 7.39 mmol), Compound 4-e (412 mg, 2.46 mmol), $K_2CO_3$ (1.02 g, 7.39 mmol), and NaI (185 mg, 1.23 mmol) were added to DCM (40 mL), followed by stirring at 80°C for 5 hours. Compound 4-e (1.03 g, 2.46 mmol) was added and stirred at 80°C for 3 hours. Afterwards, it was diluted with purified water (80 mL), extracted with DCM (80 mL×3 times), washed with brine (150 mL), and dried with $Na_2SO_4$, followed by filtration. The filtrate was concentrated under reduced pressure and purified on silica gel (PE/EtOAc = 100/1 to 3/1) to obtain Compound 4-f in a yellow oil form. Compound 4-f (400 mg, 0.474 mmol) was added to tetrahydrofuran (hereinafter referred to as THF, 10 mL), palladium/carbon catalyst (Pd/C, 80 mg, purity 10%) was added in the presence of nitrogen gas, with replacement of hydrogen, and stirring was performed in the presence of hydrogen gas (15 psi) at 25°C for 4 hours. Afterwards, the liquid filtered with DCM (20 mL) was dried and filtered with $Na_2SO_4$, concentrated under reduced pressure, and freeze-dried to obtain Compound 4 in a yellow oil form. The result of $^1$H NMR analysis for Compound 4 is as shown below.
$^1$H NMR(400MHz, $CDCl_3$) δ 7.90(d, J = 2.0Hz, 1H), 7.29(dd, J = 8.4, 2.4Hz, 1H), 6.46(d, J = 8.4Hz, 1H), 4.32(s, 2H), 4.07(t, J = 6.4Hz, 4H), 2.61-2.62(m, 4H), 2.47-2.48(m, 4H), 2.28-2.35(m, 2H), 1.56-1.66(m, 8H), 1.31-1.45(m, 16H), 1.26-1.30(m, 40H), 0.86-0.89(m, 12H)

1-5. Preparation of Compound 5

As shown in FIG. 5, Compound 5-a (4.00 g, 9.54 mmol), Compound 5-b (442 mg, 3.18 mmol), $K_2CO_3$ (1.32 g, 9.54 mmol), and NaI (238 mg, 1.59 mmol) were added to MeCN (60 mL) and stirred at 80°C for 3 hours. Compound 5-a (1.00 g, 2.38 mmol) was added, reacted by stirring at 80°C for 1 hour, and then diluted with purified water (80 mL), and a liquid obtained by extraction with DCM (80 mL×3 times) was washed with brine (150 mL) and dried with $Na_2SO_4$, followed by filtration. The filtrate was concentrated under reduced pressure and then purified on the silica gel twice (DCM/MeOH = 100/1 to 19/1, DCM (0.1% 7M $NH_3$, MeOH)/MeOH = 100/1 to 24/1) to obtain Compound 5 in a light yellow oil form. The result of $^1$H NMR analysis for Compound 5 is as shown below.

$^1$H NMR(500MHz, $CDCl_3$) δ 6.90(s, 1H), 6.82(s, 1H), 4.06(t, J = 6.5Hz, 4H), 3.87(t, J = 7.0Hz, 2H), 2.37-2.41(m,

9H), 2.28-2.34(m, 2H), 1.81-1.87(m, 2H), 1.56-1.65(m, 8H), 1.35-1.44(m, 12H), 1.25-1.30(m, 44H), 0.86-0.89(m, 12H).

1-6. Preparation of Compound 6

As shown in FIG. 6, Compound 6-a (5.00 g, 52.0 mmol) was added to THF (50 mL), and sodium hydroxide (2.08 g, 52.0 mmol) was added at 0°C after cooling, followed by stirring for 1 hour. Afterwards, Compound 6-b (9.30 g, 34.7 mmol) dissolved in THF (20 mL) was added and stirred at 25°C for 11 hours. The reaction was terminated with saturated ammonium chloride (50 mL), extracted with MeCN (80 mL×3 times), washed with brine (50 mL), and dried with $Na_2SO_4$, followed by filtration. The filtrate was concentrated under reduced pressure and purified on silica gel (DCM/MeOH = 100/1 to 16/1) to obtain Compound 6-c in a white solid form. Compound 6-c (1.50 g, 5.29 mmol) was added to ethanol (20 mL) followed by addition of hydrazine hydrate (1.03 g, 17.5 mmol), and then stirring was performed at 70°C for 2 hours. Afterwards, it was diluted with purified water (80 mL), extracted with DCM (30 mL×2 times), washed with brine (40 mL), and dried with $Na_2SO_4$, followed by fitlration. The filtered liquid was concentrated under reduced pressure to obtain Compound 6-d in a yellow oil form. Compound 6-d (584 mg, 3.82 mmol), Compound 6-e (4.00 g, 9.54 mmol), $K_2CO_3$ (1.58 g, 11.4 mmol), and NaI (286 mg, 1.91 mmol) were added to MeCN (60 mL) and stirred at 80°C for 4 hours. Compound 6-e (1.60 g, 3.82 mmol) and $K_2CO_3$ (527 mg, 3.82 mmol) were added and stirred at 80°C for 3 hours to complete the reaction. Afterwards, it was diluted with purified water (100 mL), extracted with DCM (80 mL×3 times), washed with brine (150 mL), and dried with $Na_2SO_4$, followed by filtration. The filtrate was concentrated under reduced pressure and purified on silica gel (DCM/MeOH = 100/1 to 19/1) to obtain Compound 6 in a yellow oil form. The result of $^1$H NMR analysis for Compound 6 is as shown below.

$^1$H NMR(400MHz, $CDCl_3$) δ 6.63 (s, 1H), 4.06(t, J = 6.8Hz, 4H), 3.89(t, J = 7.2Hz, 2H), 2.45-2.54(m, 9H), 2.27-2.34(m, 2H), 2.22(s, 3H), 1.90-1.97(m, 2H), 1.56-1.65(m, 8H), 1.31-1.47(m, 20H), 1.25-1.26(m, 36H), 0.86-0.89(m, 12H).

1-7. Preparation of Compound 7

As shown in FIG. 7, Compound 7-a (3.00 g, 5.62 mmol), Compound 7-b (654 mg, 1.87 mmol), $K_2CO_3$ (397 mg, 3.75 mmol), and NaI (281 mg, 1.87 mmol) were added to MeCN (25 mL) followed by replacement with nitrogen gas, and then the mixture was stirred at 50°C in the presence of nitrogen gas for 4 hours. Afterwards, it was diluted with purified water (60 mL), extracted with EtOAc (50 mL×3 times), washed with brine (100 mL), and dried with $Na_2SO_4$, followed by filtration. The filtrate was concentrated under reduced pressure, and purified on the silica gel twice (DCM/MeOH = 100/1 to 19/1 and DCM (0.1% 7M ammonia water/MeOH)/MeOH = 100/1 to 10/1) to obtain Compound 7 in a yellow oil form. The result of $^1$H NMR analysis for Compound 7 is as shown below.

$^1$H NMR(400MHz, $CDCl_3$) δ 6.54(s, 1H), 4.84-4.90(m 1H), 4.06(t, J = 6.8Hz, 2H), 3.82(t, J = 6.8, 2H), 2.39-2.42(m, 6H), 2.35-2.36(m, 3H), 2.28-2.30(m, 4H), 2.18(s, 3H), 1.82-1.84(m, 2H), 1.57-1.67(m, 6H), 1.50-1.55(m, 4H), 1.40-1.44(m, 4H), 1.24-1.35(m, 48H), 0.86-0.70(m, 9H).

1-8. Preparation of Compound 8

As shown in FIG. 8, Compound 8-a (3.00 g, 5.62 mmol), Compound 8-b (549 mg, 1.87 mmol), $Na_2CO_3$ (397 mg, 3.75 mmol), and NaI (281 mg, 1.87 mmol) were added to MeCN (25 mL) followed by replacement with nitrogen gas, and then stirring was performed at 50°C in the presence of nitrogen gas for 4 hours. Afterwards, it was diluted with purified water (60 mL), extracted with EtOAc (50 mL×3 times), washed with brine (100 mL), and dried with $Na_2SO_4$, followed by filtration. The filtrate was concentrated under reduced pressure and purified on the silica gel twice (DCM/MeOH = 100/1 to 19/1 and DCM (0.1% 7M $NH_3$.MeOH)/MeOH = 100/1 to 10/1) to obtained Compound 8 in a yellow oil form. The result of $^1$H NMR analysis for Compound 8 is as shown below.

$^1$H NMR(400MHz, $CDCl_3$) δ 6.54(s, 1 H), 4.84-4.94(m, 2H), 3.82(t, J = 7.2Hz, 2H), 2.39-2.42(m, 6H), 2.37(s, 3H), 2.25-2.29(m, 4H), 2.18(s, 3H), 1.82-1.84(m, 2H), 1.57-1.62(m, 6H), 1.51-1.55(m, 4H), 1.39-1.42(m, 4H), 1.26-1.32(m, 38H), 1.20(d, J = 6.0Hz, 3H), 0.87-0.92(m, 9H).

[Example 2] Characterization of the size and surface charge of lipid nanoparticles

The size and surface charge of lipid nanoparticles according to the ionizable lipid prepared in Example 1 were measured. To measure the size of the lipid nanoparticles, dilution was conducted using PBS to reach the concentration of mRNA contained in each lipid nanoparticle to 1 μg/mL, and Dynamic Light Scattering (DLS) of Mlvern Zetasizer Nano (Malvern Instruments, UK) was applied to measure the diameter and a polydispersity index (hereinafter referred to as PDI) of lipid nanoparticles. In addition, in order to measure the surface charge of lipid nanoparticles, dilution was conducted using PBS to reach the concentration of mRNA contained in each lipid nanoparticle to 1 μg/mL, and the surface charge

(zeta potential) was measured using the same equipment as above.

As a result, as shown in Table 1, the lipid nanoparticles all showed a size of 80~120 nm and optimum PDI, while the surface charge showed a value of +30mV or less in water.

TABLE 1

| Compound Number | Particle size (nm) | PDI | Surface Charge (mV) |
|---|---|---|---|
| 1 | 121 | 0.09 | +29.1 |
| 2 | 106 | 0.12 | +28.1 |
| 3 | 78 | 0.18 | -1.4 |
| 4 | 84 | 0.13 | +2.0 |
| 5 | 104 | 0.08 | +19.9 |
| 6 | 102 | 0.07 | +21.1 |
| 7 | 109 | 0.24 | +26.3 |

[Example 3] Determination of a gene encapsulation efficiency

To determine a gene encapsulation efficiency of lipid nanoparticles, the Quant-iT™ Ribogreen™ RNA Reagent and Kit was used. A stock solution for the calibration curve was prepared by taking 5 μL of the ribosomal RNA standard (100 μg/mL in TE buffer) and diluting it with either 245 μL of TE buffer or 0.4% Triton-TE buffer, respectively. Aliquots of 2, 5, 10, 25, and 50 μL of the stock solution were taken, diluted with TE buffer or 0.4% Triton-TE buffer to achieve a total volume of 100 μL, and then mixed with 100 μL of Quant-iT™ Ribogreen™ RNA Reagent; the fluorescence intensity (excitation 475 nm and emission 500~550 nm) was measured to create a calibration curve. A volume of 5 μL of the nanoparticle solution prepared in Example 2 was taken and diluted with 245 μL of TE buffer (10 mM Tris-HCl, 1 mM EDTA, pH 7.5 in DEPC-treated water). After 50 μL of TE buffer or 50 μL of 2% Triton-TE buffer was added to 50 μL of the diluted nanoparticle solution, respectively, the mixture was incubated at 37°C for about 10 minutes. Afterwards, 40 μL of each mixture was transferred to a microplate, and 60 μL of TE buffer and 100 μL of Quant-iT™ Ribogreen™ RNA Reagent were added sequentially to measure the fluorescence intensity. After calculating the gene dosage (T0) in the TE buffer and the gene dosage (T2) in the 2% Triton-TE buffer by substituting the measured fluorescence intensity into the calibration curve, the gene encapsulation efficiency was calculated using the following Mathematical Equation 1. The values of gene encapsulation efficiency were listed in Table 2. As a result, all the compounds showed optimal gene encapsulation efficiency.

[Mathematical Equation 1]

$$\textit{Gene encapsulation efficiency (\%)} = \frac{(T2\text{-}T0)}{T2} * 100$$

As a result, as shown in Table 2, it was found that all the compounds exhibited favorable gene encapsulation efficiency.

TABLE 2

| Compound Number | Gene encapsulation efficiency (%) |
|---|---|
| 1 | 99.7 |
| 2 | 94.9 |
| 3 | 93.8 |
| 4 | 78.2 |
| 5 | 93.5 |
| 6 | 94.1 |
| 7 | 98.9 |

[Example 4] Evaluation of the gene delivery efficacy of lipid nanoparticles

4-1. Evaluation of the intracellular gene delivery efficacy

To determine the intracellular gene delivery efficacy of lipid nanoparticles, lipid nanoparticles encapsulated with FLuc mRNA(Fluc mRNA-LNP) were used. To evaluate the gene delivery efficacy, HeLa cells (Korea Cell Line Bank, Seoul), human cervical cancer cell lines expressing low-density lipoprotein (LDL) receptors on the cell surface, were used, and cultured with the MEM medium containing 10% fetal bovine serum and penicillin/streptomycin at 37°C in the presence of 5% $CO_2$. HeLa cell lines were seeded at $2\times10^4$ cells per well in a 96-well plate, and after 16~24 hours, the cells were treated with lipid nanoparticles at a concentration of 0.25 μ g/ml based on encapsulated mRNA, in a total volume of 100 μl MEM medium per well in the absence or the presence of 10% fetal bovine serum (FBS).

To measure the expression of intracellular luciferase after FLuc mRNA delivery by lipid nanoparticles, the cells were washed with PBS after 6 hours of treatment. Then, and 100 μl of Glo-Lysis Buffer (Promega, WI, USA) was added to lyse the cells at room temperature for 5 minutes. The lysed cells were added with 100 μl of Steady-Glo™ Luciferase Assay solution (Promega) in a 1:1 ratio, left at room temperature for 5 minutes, and then transferred to a 96-well white plate. Luminescent values were detected using a GloMax Discover microplate reader (Promega). Recombinant luciferase (Promega) was used as the standard material to generate the calibration curve, which was used to determine the luciferase concentration (pg/ml) in the samples.

As shown in Table 3, it was determined that lipid nanoparticles (Compounds 1-2) showed a high gene delivery efficacy even in the absence of FBS. These results indicated that the lipid nanoparticles could be effectively utilized for gene delivery even in a serum-free environment.

TABLE 3

| | Luciferase expression level (Mean±SD, pg/ml) | |
|---|---|---|
| Compound Number | w/o FBS | 10% FBS |
| 1 | 16,685±646 | 16,060±102 |
| 2 | 15,270±2,273 | 8,492±2,375 |

4-2. Evaluation of the gene delivery efficiency in vivo

In order to identify the gene delivery efficacy and distribution of lipid nanoparticles in vivo, lipid nanoparticles were injected into 7-week-old mice of the C57BL/6 lineage either intravenously or intramuscularly in the thigh. The liver was extracted in the case of intravenous injection and the organs including the femoral muscle were extracted in the case of intramuscular injection by sacrificing the mice after 4 hours.

To determine the level of luciferase expression in tissue samples, the tissue sections were weighed and transferred to a tube containing 3 mm metal beads, and a homogenized by adding 500 μl of Glo-Lysis Buffer (Promega) per 50 mg of tissue. The supernatant from the centrifuged homogenates was collected and diluted with the Glo-Lysis Buffer to an appropriate ratio. The diluted homogenates and the Steady-Glo Luciferase Assay solution were then mixed in a 1:1 ratio, with 50 μl of each. After a 5-minute incubation, the luminescence values of luciferase were measured using the same method as described in Example 3-1, and the expression levels (ng/g tissue) of luciferase were calculated.

As a result, as shown in Table 4, the lipid nanoparticles (Compounds 4-6) demonstrated favorable gene delivery efficacy to the liver following intravenous injection, and as shown in Table 5, the lipid nanoparticles (Compounds 5-6) showed locally restricted gene delivery in the muscle when injected intramuscularly.

TABLE 4

| Compound Number | Luciferase expression level (Mean±SD, ng/g) |
|---|---|
| 4 | 6,844±2,052 |
| 5 | 2,582±745 |
| 6 | 1,172±99 |

TABLE 5

| Compound Number | Luciferase expression level (Mean±SD, ng/g) |
|---|---|
| 5 | 651±158 |
| 6 | 358±74 |

While a specific part of the present invention has been described in detail above, it is clear for those skilled in the art that this specific description is merely preferred example embodiments, and the scope of the present invention is not limited thereby. In other words, the substantial scope of the present invention is defined by the appended claims and their equivalents.

## Claims

1. A compound selected from a compound represented by the following Chemical Formula I, or a pharmaceutically acceptable salt, tautomer, prodrug, or stereoisomer thereof:

[Chemical Formula I]

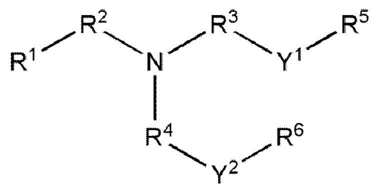

wherein, in the Chemical Formula I,

the $R^1$ is selected from the group consisting of the following Chemical Formula I-1 to Chemical Formula I-3,

[Chemical Formula I-1]

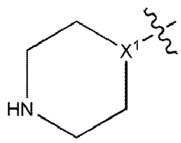

[Chemical Formula I-2]

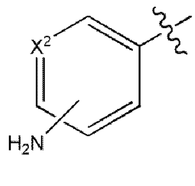

[Chemical Formula I-3]

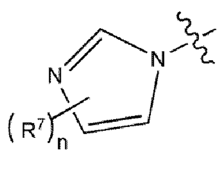

the $R^2$ is selected from alkyl, alkenyl, or alkynyl of substituted or unsubstituted $C_{1-6}$ wherein the substitution is such that at least one $-CH_2-$ is substituted with one selected from the group consisting of -O-, -C(=O)-, -OC(=O)-, -C(=O)O-, -OC(=O)O-, $-OP(=O)(OR^2)O-$, - OP(=O)(OH)O-, -OC(=S)-, -C(=S)O-, -SC(=O)-, -C(=O)S-, -SC(=S)-, -C(=S)S-, -SC(=O)O-, -OC(=O)S-, -SS-, -C(=O)NH-, -NHC(=O)-, $-C(=O)NR^2-$, $-NR^2C(=O)-$, -OC(=O)NH-, - NHC(=O)O-, $-OC(=O)NR^2-$, and $-NR^2C(=O)O-$,
the $R^3$ and $R^4$ are independently selected from alkyl, alkenyl, or alkynyl of substituted or unsubstituted $C_{2-10}$ wherein the substitution is such that at least one $-CH_2-$ is substituted with -CH(OH)-, -O-, or -SS-,
the $R^5$ and $R^6$ are independently selected from alkyl, alkenyl, alkynyl, cycloalkyl, cycloalkenyl, cycloalkynyl, heterocyclyl, aryl, or heteroaryl of substituted or unsubstituted branched $C_{3-20}$ wherein the substitution is such that at least one $-CH_2-$ is substituted with - CH(OH)-, -O-, or -SS-,
the $Y^1$ and $Y^2$ are selected from -OC(=O)- or -C(=O)O-,
the $X^1$ and $X^2$ are selected from CH or N,
the $R^7$ is alkyl of $C_{1-6}$, and
n is an integer of 1 to 2.

**2.** The compound of claim 1, wherein the $R^1$ is selected from the group consisting of the following Chemical Formulas I-4 to I-9:

[Chemical Formula I-4]

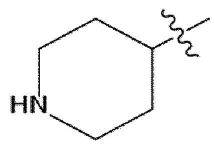

[Chemical Formula I-5]

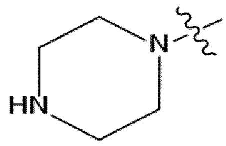

[Chemical Formula I-6]

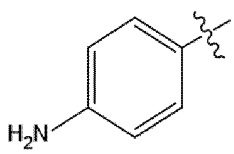

[Chemical Formula I-7]

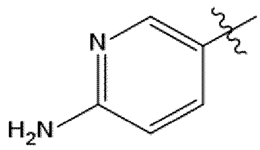

[Chemical Formula I-8]

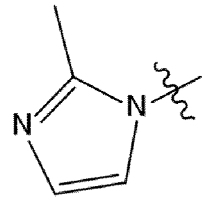

[Chemical Formula I-9]

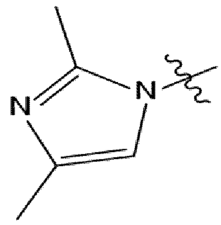

.

3. The compound of claim 1, wherein the $R^2$ is alkyl of substituted or unsubstituted $C_{1-6}$ wherein the substitution is such that at least one $-CH_2-$ is substituted with one selected from the group consisting of -O-, -C(=O)-, -OC(=O)-, -C(=O)O-, and -SS-.

4. The compound of claim 1, wherein the $R^3$ and $R^4$ are independently alkyl of substituted or unsubstituted $C_{2-10}$ wherein the substitution is such that at least one $-CH_2-$ is substituted with -CH(OH)-, -O-, or -SS-.

5. The compound of claim 1, wherein the $R^5$ and $R^6$ are independently alkyl or alkenyl of substituted or unsubstituted branched $C_{3-20}$ wherein the substitution is such that at least one $-CH_2-$ is substituted with -CH(OH)-, -O-, or -SS-.

6. The compound of claim 1, wherein the $R^1$ is selected from the group consisting of the following Chemical Formulars I-4 to I-9, $R^2$ is alkyl of substituted or unsubstituted $C_{1-6}$ wherein the substitution is such that at least one $-CH_2-$ is substituted with one selected from the group consisting of -O-, -C(=O)-, -OC(=O)-, -C(=O)O-, and -SS-, the $R^3$ and $R^4$ are independently alkyl of substituted or unsubstituted $C_{2-10}$ wherein the substitution is such that at least one $-CH_2-$ is substituted with -CH(OH)-, -O-, or -SS-, and the $R^5$ and $R^6$ are independently alkyl or alkenyl of substituted or unsubstituted branched $C_{3-20}$ wherein the substitution is such that at least one $-CH_2-$ is substituted with -CH(OH)-, -O-, or - SS-:

[Chemical Formula I-4]

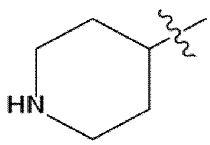

[Chemical Formula I-5]

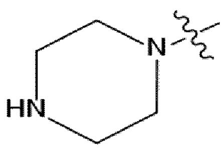

[Chemical Formula I-6]

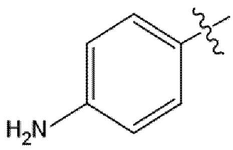

[Chemical Formula I-7]

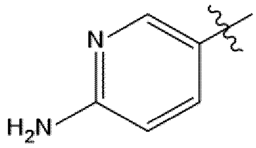

[Chemical Formula I-8]

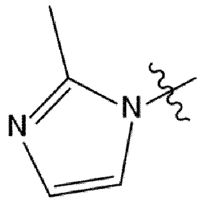

[Chemical Formula I-9]

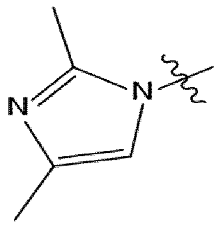

.

**7.** The compound of claim 1, wherein the compound is one or more selected from the group consisting of ((2-(piperidin-4-yl)ethyl)azanediyl)bis(hexane-6,1-diyl) bis(2-hexyldecanoate); ((2-(piperazin-1-yl)ethyl)azanediyl)bis(hexane-6,1-diyl) bis(2-hexyldecanoate); ((4-aminobenzyl)azanediyl)bis(hexane-6,1-diyl) bis(2-hexyldecanoate); ((2-(6-aminopyridin-3-yl)ethyl)azanediyl)bis(hexane-6,1-diyl) bis(2-hexyldecanoate); ((3-(2-methyl-1H-imidazol-1-yl)propyl)azanediyl)bis(hexane-6,1-diyl) bis(2-hexyldecanoate); ((3-(2,4-dimethyl-1H-imidazol-1-yl)propyl)azanediyl)bis(hexane-6,1-diyl)bis(2-hexyldecanoate); heptadecan-9-yl 8-((3-(2,4-dimethyl-1H-imidazol-1-yl)propyl)(8-(nonyloxy)-8-oxooctyl)amino)octanoate; bis(2-hexyldecyl) 7,7'-((2-(6-aminopyridin-3-yl)ethyl)azanediyl) diheptanoate; ((2-(6-aminopyridin-3-yl)ethyl)azanediyl)bis(hexane-6,1-diyl) bis(2-(2-(butyldisulfanyl)ethyl)octanoate); ((((3-(2-methyl-1H-imidazol-1-yl)propyl)azanediyl)bis(ethane-2,1-diyl))bis(oxy))bis(propane-3,1-diyl)bis(2-hexyldecanoate); and ((3-((2,4-dimethyl-1H-imidazol-1-yl)methoxy)propyl)azanediyl) bis(hexane-6,1-diyl) bis(2-hexyldecanoate).

**8.** A lipid nanoparticle composition comprising the compound according to any one of claims 1 to 7.

**9.** The lipid nanoparticle composition of claim 8, wherein the compound is an ionizable lipid.

**10.** The lipid nanoparticle composition of claim 9, wherein the composition further comprises one or more selected from the group consisting of a neutral lipid, steroid, and polymeric polylipid.

**11.** The lipid nanoparticle composition of claim 10, wherein the neutral lipid is a phospholipid or glycolipid.

**12.** The lipid nanoparticle composition of claim 10, wherein the steroid is one or more selected from the group consisting of cholesterol, bile acid derivatives, and cholinic acid derivatives.

**13.** The lipid nanoparticle composition of claim 10, wherein the polymeric polylipid is a pegylated lipid and is one or more selected from the group consisting of PEG-modified phosphatidylethanolamine, PEG-modified phosphatidic acid, PEG-modified ceramide, PEG-modified dialkylamine, PEG-modified diasylglycerol, and PEG-modified dialkylglycerol.

**14.** The lipid nanoparticle composition of claim 10, wherein the composition comprises 20 to 65 mol% of the ionizable lipid, 2.5 to 30 mol% of the neutral lipid, 25 to 60 mol% of the steroid, and 0.5 to 5 mol% of the polymeric polylipid.

**15.** The lipid nanoparticle composition of claim 8, wherein the composition further comprises a prophylactic or therapeutic agent.

**16.** The lipid nanoparticle composition of claim 15, wherein the prophylactic or therapeutic agent is one or more selected from the group consisting of small interfering RNA (siRNA), ribosomal ribonucleic acid (rRNA), ribonucleic acid (RNA), deoxyribonucleic acid (DNA), complementary deoxyribonucleic acid (cDNA), aptamer, messenger ribonucleic acid (mRNA), transfer ribonucleic acid (tRNA), anti-sense oligonucleotide, small hairpin ribonucleic acid (shRNA), microribonucleic acid (miRNA), asymmetric interfering ribonucleic acid (aiRNA), dicer-substrate ribonucleic acid (dsRNA), ribozyme, peptide nucleic acid (PNA), deoxyribozyme (DNAzyme), guide ribonucleic acid (sgRNA) for gene editing, and a mixture thereof.

**17.** A composition for drug delivery, comprising the lipid nanoparticle composition according to claim 8; and a prophylactic or therapeutic agent.

[FIG. 1]

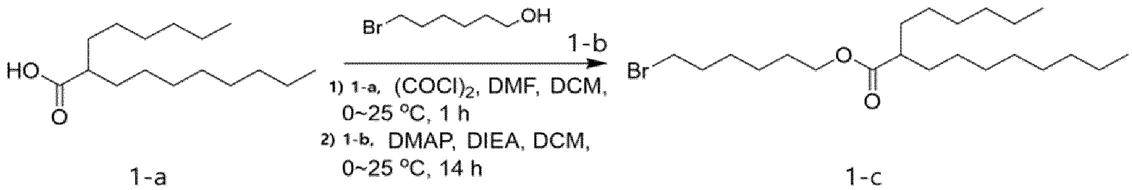


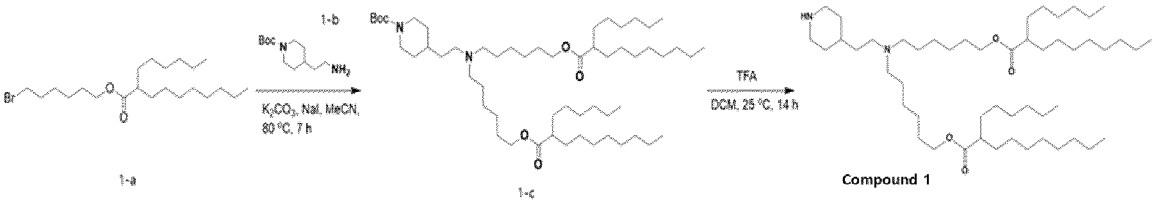


[FIG. 2]

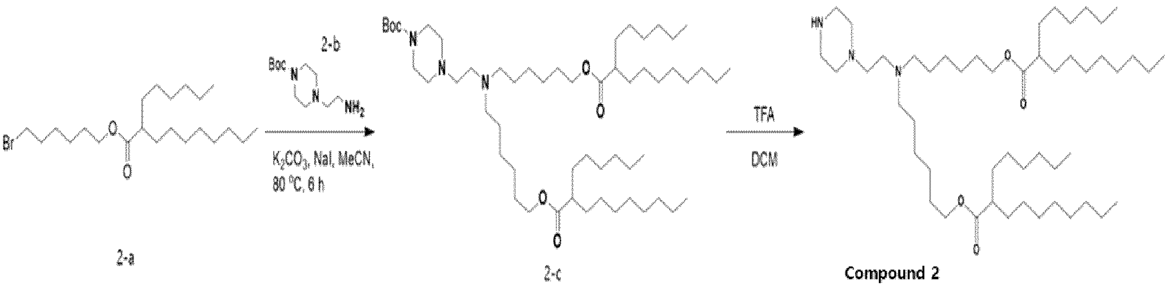


[FIG. 3]

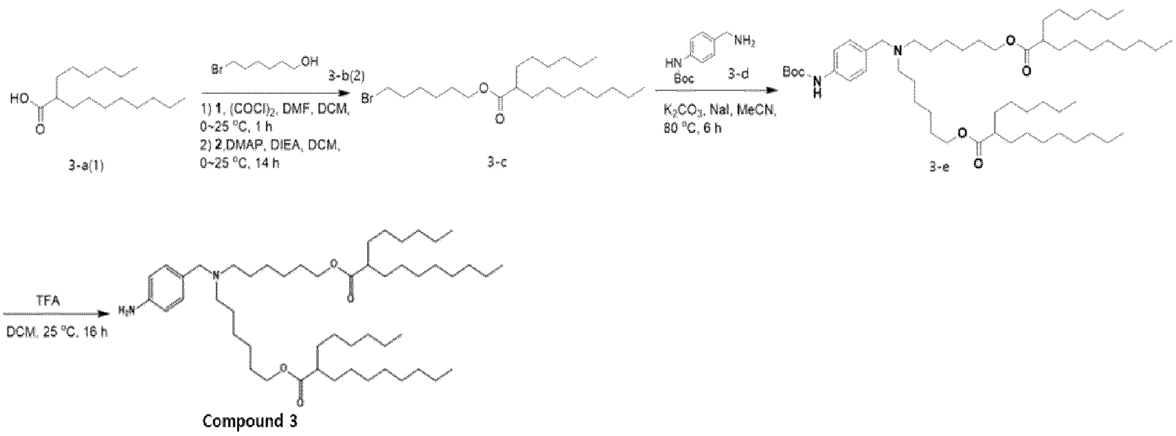

[FIG. 4]

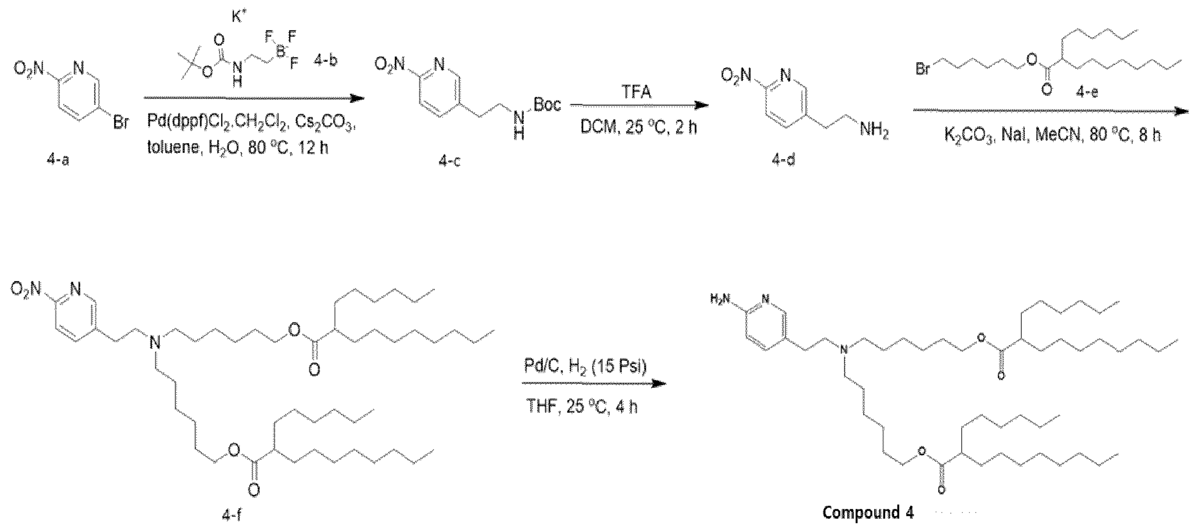


[FIG. 5]

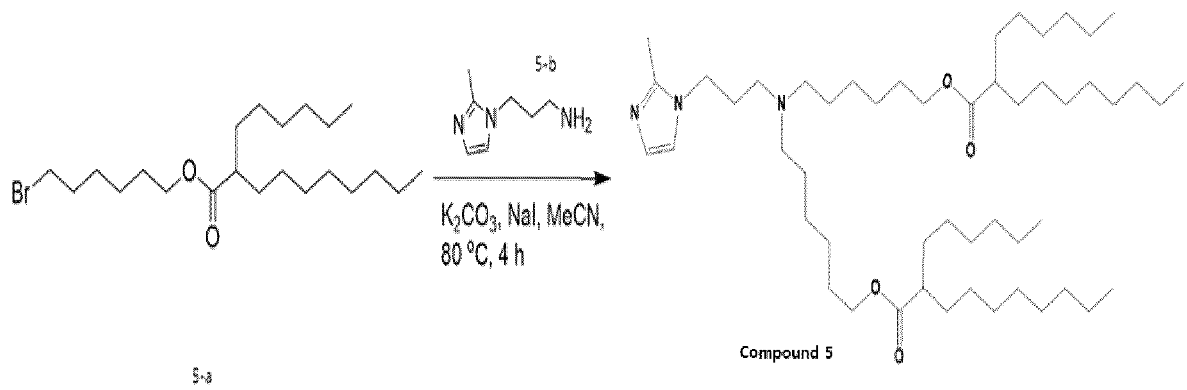


[FIG. 6]

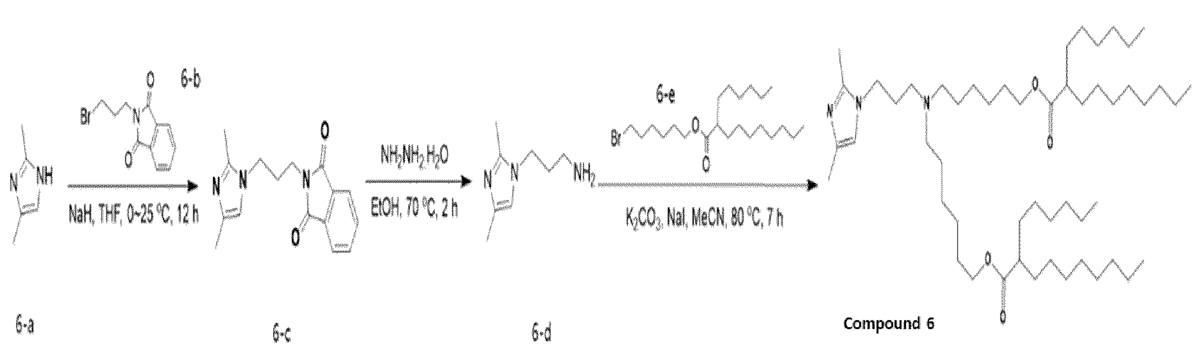

[FIG. 7]

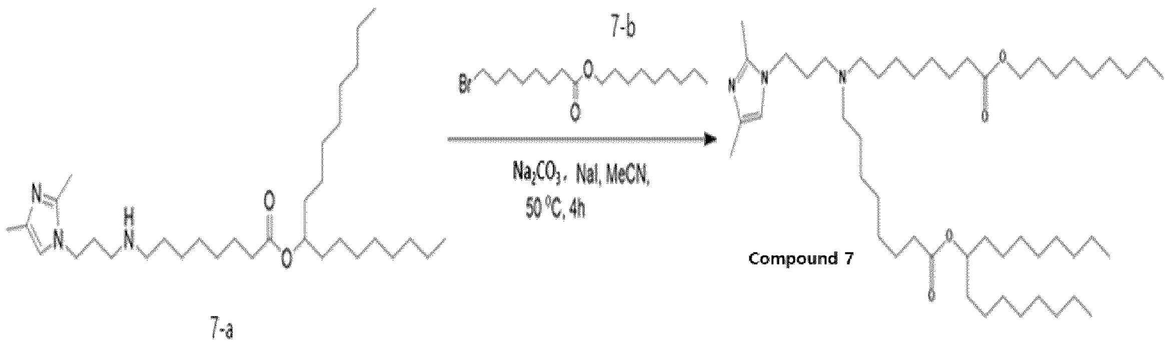


7-a

[FIG. 8]

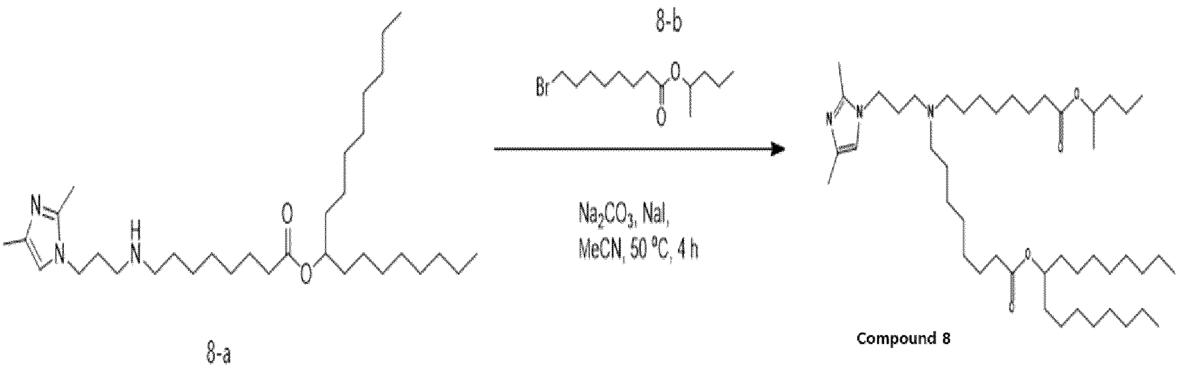


8-a

**INTERNATIONAL SEARCH REPORT**

International application No.
**PCT/KR2023/003696**

**A. CLASSIFICATION OF SUBJECT MATTER**

**C07D 211/26**(2006.01)i; **A61K 9/51**(2006.01)i; **A61K 9/127**(2006.01)i; **A61K 47/22**(2006.01)i; **A61K 47/18**(2006.01)i; **C07D 295/13**(2006.01)i; **C07D 211/56**(2006.01)i; **C07D 233/61**(2006.01)i; **C07D 213/73**(2006.01)i; **C07D 233/60**(2006.01)i

According to International Patent Classification (IPC) or to both national classification and IPC

**B. FIELDS SEARCHED**

Minimum documentation searched (classification system followed by classification symbols)

C07D 211/26(2006.01); A61K 31/7088(2006.01); A61K 48/00(2006.01); C07C 229/12(2006.01); C07C 229/16(2006.01); C07C 229/24(2006.01); C07C 233/36(2006.01); C07C 235/10(2006.01); C07C 311/05(2006.01); C07C 311/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 약물 전달(drug delivery), 이온화 지질(ionized lipid), 지질나노입자(lipid nanoparticles), 아민 화합물(amine compound)

**C. DOCUMENTS CONSIDERED TO BE RELEVANT**

| Category* | Citation of document, with indication, where appropriate, of the relevant passages | Relevant to claim No. |
|---|---|---|
| X | WO 2021-226597 A2 (ORNA THERAPEUTICS, INC.) 11 November 2021 (2021-11-11)<br>See claims 1, 10 and 102; and pages 2, 4, 240 (table 10a) and 665 (example 70B). | 1-17 |
| X | CN 113185421 A (GUANGZHOU RIBOBIO CO., LTD.) 30 July 2021 (2021-07-30)<br>See claims 1, 22-31 and 33; and paragraph [0080] (compound A44). | 1-17 |
| A | WO 2021-204175 A1 (SUZHOU ABOGEN BIOSCIENCES CO., LTD.) 14 October 2021 (2021-10-14)<br>See entire document. | 1-17 |
| A | WO 2021-055849 A1 (MODERNATX, INC.) 25 March 2021 (2021-03-25)<br>See entire document. | 1-17 |
| A | WO 2018-200943 A1 (ACUITAS THERAPEUTICS, INC.) 01 November 2018 (2018-11-01)<br>See entire document. | 1-17 |

☐ Further documents are listed in the continuation of Box C.  ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 July 2023** | **07 July 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2023/003696**

| Patent document cited in search report | Publication date (day/month/year) | Patent family member(s) | Publication date (day/month/year) |
|---|---|---|---|
| WO 2021-226597 A2 | 11 November 2021 | AU 2019-280583 A1 | 03 December 2020 |
| | | AU 2019-280583 B2 | 15 December 2022 |
| | | AU 2020-280105 A1 | 20 January 2022 |
| | | AU 2020-397956 A1 | 07 July 2022 |
| | | BR 112020024292 A2 | 02 March 2021 |
| | | BR 112021023411 A2 | 01 February 2022 |
| | | CA 3178111 A1 | 11 November 2021 |
| | | CN 116113419 A | 12 May 2023 |
| | | EP 3801639 A1 | 14 April 2021 |
| | | EP 3920976 A2 | 15 December 2021 |
| | | EP 3972653 A1 | 30 March 2022 |
| | | EP 4146285 A2 | 15 March 2023 |
| | | EP 4153244 A1 | 29 March 2023 |
| | | IL 279157 A | 31 January 2021 |
| | | JP 2021-526792 A | 11 October 2021 |
| | | JP 2022-533796 A | 25 July 2022 |
| | | JP 2023-504568 A | 03 February 2023 |
| | | JP 2023-525270 A | 15 June 2023 |
| | | KR 10-2021-0018323 A | 17 February 2021 |
| | | KR 10-2022-0027855 A | 08 March 2022 |
| | | MX 2020013236 A | 22 February 2021 |
| | | SG 11202011162 A | 30 December 2020 |
| | | US 11203767 B2 | 21 December 2021 |
| | | US 11352640 B2 | 07 June 2022 |
| | | US 11352641 B2 | 07 June 2022 |
| | | US 11447796 B2 | 20 September 2022 |
| | | US 11603396 B2 | 14 March 2023 |
| | | US 2021-0198688 A1 | 01 July 2021 |
| | | US 2021-0363540 A1 | 25 November 2021 |
| | | US 2021-0403944 A1 | 30 December 2021 |
| | | US 2022-0025395 A1 | 27 January 2022 |
| | | US 2022-0177540 A1 | 09 June 2022 |
| | | US 2022-0323480 A1 | 13 October 2022 |
| | | US 2023-0058784 A1 | 23 February 2023 |
| | | US 2023-0062665 A1 | 02 March 2023 |
| | | WO 2019-236673 A1 | 12 December 2019 |
| | | WO 2020-237227 A1 | 26 November 2020 |
| | | WO 2021-113777 A2 | 10 June 2021 |
| | | WO 2021-113777 A3 | 02 September 2021 |
| | | WO 2021-226597 A3 | 03 March 2022 |
| | | WO 2021-236855 A1 | 25 November 2021 |
| CN 113185421 A | 30 July 2021 | AU 2021-245162 A1 | 16 June 2022 |
| | | CA 3133528 A1 | 27 May 2022 |
| | | CN 113185421 B | 25 January 2022 |
| | | EP 4025556 A1 | 13 July 2022 |
| | | IL 287069 A | 01 December 2021 |
| | | JP 2023-514951 A | 12 April 2023 |
| | | KR 10-2023-0042716 A | 29 March 2023 |
| | | TW 202248190 A | 16 December 2022 |
| | | WO 2022-112855 A1 | 02 June 2022 |

Form PCT/ISA/210 (patent family annex) (July 2022)

**INTERNATIONAL SEARCH REPORT**
**Information on patent family members**

International application No.
**PCT/KR2023/003696**

| Patent document cited in search report | Publication date (day/month/year) | Patent family member(s) | Publication date (day/month/year) |
|---|---|---|---|
| | | ZA 202108087 B | 31 August 2022 |
| WO 2021-204175 A1 | 14 October 2021 | AU 2021-254312 A1 | 27 October 2022 |
| | | BR 112022020203 A2 | 22 November 2022 |
| | | CA 3178455 A1 | 14 October 2021 |
| | | CN 114206827 A | 18 March 2022 |
| | | CN 114206827 B | 23 May 2023 |
| | | EP 4077272 A1 | 26 October 2022 |
| | | IL 297084 A | 01 December 2022 |
| | | KR 10-2022-0166802 A | 19 December 2022 |
| | | TW 202204309 A | 01 February 2022 |
| | | US 11472766 B2 | 18 October 2022 |
| | | US 2022-0153686 A1 | 19 May 2022 |
| | | ZA 202210782 B | 26 April 2023 |
| WO 2021-055849 A1 | 25 March 2021 | AU 2020-351225 A1 | 07 April 2022 |
| | | BR 112022004771 A2 | 21 June 2022 |
| | | CA 3154720 A1 | 25 March 2021 |
| | | CN 114746398 A | 12 July 2022 |
| | | EP 4031527 A1 | 27 July 2022 |
| | | IL 291375 A | 01 May 2022 |
| | | JP 2022-548312 A | 17 November 2022 |
| | | KR 10-2022-0103923 A | 25 July 2022 |
| | | US 2023-0000773 A1 | 05 January 2023 |
| WO 2018-200943 A1 | 01 November 2018 | AU 2018-256877 A1 | 21 November 2019 |
| | | AU 2018-256877 B2 | 02 June 2022 |
| | | AU 2022-224779 A1 | 27 October 2022 |
| | | CA 3061612 A1 | 01 November 2018 |
| | | CN 110799492 A | 14 February 2020 |
| | | EP 3615510 A1 | 04 March 2020 |
| | | JP 2020-517694 A | 18 June 2020 |
| | | US 11357856 B2 | 14 June 2022 |
| | | US 2020-0046838 A1 | 13 February 2020 |
| | | US 2022-0081392 A1 | 17 March 2022 |
| | | WO 2018-191657 A1 | 18 October 2018 |
| | | WO 2018-191719 A1 | 18 October 2018 |

Form PCT/ISA/210 (patent family annex) (July 2022)